# EUROPEAN PATENT SPECIFICATION

(11) **EP 3 936 101 B1**
(45) Date of publication and mention of the grant of the patent: **05.08.2026**
(21) Application number: 20184383.6
(22) Date of filing: 07.07.2020
(51) Int. Cl.: A61F 13/42

(54) **IDENTIFICATION DEVICE, USAGE METHOD, ARRAY AND IDENTIFICATION SYSTEM FOR AN ABSORBENT ARTICLE**
IDENTIFIKATIONSVORRICHTUNG, VERWENDUNGSVERFAHREN, ARRAY UND IDENTIFIKATIONSSYSTEM FÜR EINEN SAUGFÄHIGEN ARTIKEL
DISPOSITIF D'IDENTIFICATION, PROCÉDÉ D'UTILISATION, RÉSEAU ET SYSTÈME D'IDENTIFICATION D'ARTICLE ABSORBANT

(43) Date of publication of application: 12.01.2022
(73) Proprietor: Ontex BV, 9255 Buggenhout (BE); Ontex Group NV, 9320 Erembodegem (BE)
(72) Inventor: HELLMOLD, Jens, 59269 Beckum (DE); HEIRMAN, Lisa, 9250 Waasmunster (BE); GIAUFER, Joël, 31750 Escalquens (FR); HERMANS, Bart, 1860 Meise (BE); VANHELMOLT, Niels, 3300 Tienen (BE)
(74) Representative: Saurat, Thibault

(56) References cited:
- WO-A1-2007/069968
- WO-A1-2008/130298
- WO-A1-2014/177200
- WO-A1-2014/177203
- US-B1- 9 233 030

## Description

The invention relates to an identification device for an absorbent article, exemplarily a diaper, of a wearer, an identification system for an absorbent article of a wearer, a usage method for using an identification device for an absorbent article of a wearer, and an array of identification devices for absorbent articles of wearers. More specifically, it is noted that the identification device may especially be attachable to the absorbent article, exemplarily the diaper, and/or said identification may especially comprise identifying the wearer of the absorbent article, exemplarily the diaper.

Generally, in times of an increasing number of elderly and care-dependent people, there is a growing need of an identification device for an absorbent article of a wearer, an identification system for an absorbent article of a wearer, a usage method for using an identification device for an absorbent article of a wearer, and an array of identification devices for absorbent articles of wearers in order to handle incontinence in a particularly efficient and flexible manner.

WO 2014/177200 A1 relates to a data capture and management system for providing an absorbent article, such as a diaper, a sanitary towel, an incontinence garment, a medical dressing and the like, with sensing and/or data logging capabilities. The system includes a data logger configured to be detachably attached to or located in the vicinity of an absorbent article, a data link between the data logger and a data collection hub, and a user interface for permitting an operator to access the data collection hub from a remote location. For ease of identification, each data logger is provided with a user-friendly identifier, and the data collection hub includes correlation means for correlating the user-friendly identifier of the data logger with an IMEI (International Mobile Station Equipment Identity) number or other unique device identifier of that data logger. Disadvantageously, especially due to said fixedly provided user-friendly identifier, flexibility cannot be ensured, which leads to inefficiencies.

Accordingly, there is an object to provide an identification device for an absorbent article of a wearer, an identification system for an absorbent article of a wearer, a usage method for using an identification device for an absorbent article of a wearer, and an array of identification devices for absorbent articles of wearers, whereby a particularly high flexibility and efficiency are ensured.

This object is solved by the features of claim 1 for an identification device for an absorbent article of a wearer, the features of claim 13 for an identification system for an absorbent article of a wearer, the features of claim 14 for a usage method for using an identification device for an absorbent article of a wearer, and the features of claim 15 for an array of identification devices for absorbent articles of wearers. The dependent claims contain further developments.

The invention is as defined in the Claims.

According to a first aspect of the invention, an identification device for an absorbent article of a wearer is provided. The identification device comprises a device identifier, a communication unit, and a pairing unit connected to the communication unit. In this context, the communication unit is configured to receive a personal identifier with respect to the wearer from a nearby back-end unit and to provide the personal identifier for the . In addition to this, the pairing unit is configured to link the personal identifier to the device identifier. Advantageously, both a particularly high flexibility and a particularly high efficiency are ensured.

Further advantageously and preferably, the identification device may provide sensing capabilities with respect to the absorbent article and/or the wearer. Additionally or alternatively, the identification device may comprise a monitoring unit being configured to monitor at least one state of the absorbent article such as wetness and/or exudates inside the absorbent article and/or at least one state with respect to the wearer such as a fall incident.

As a further advantage, the communication unit may comprise first communication means for short-range or short-distance communication and second communication means for long-range or long-distance communication. In this context, the first communication means may be configured to communicate with the nearby back-end unit. Additionally or alternatively, the second communication means may be configured to communicate with other units especially without the nearby back-end unit. Further additionally or further alternatively, the second communication means may be configured to have no access to the personal identifier. Advantageously, the short-range or short-distance communication comprises a range from immediate proximity to 150 meters, preferably to 100 meters, more preferably to 50 meters, most preferably to 20 meters. Accordingly, the first communication means may advantageously be configured to range from immediate proximity to 150 meters, preferably to 100 meters, more preferably to 50 meters, most preferably to 20 meters.

According to a first preferred implementation form of the first aspect of the invention, the personal identifier is linked to the device identifier especially on the side of the identification device. In addition to this or as an alternative, the identification device is attached to the absorbent article. Further additionally or further alternatively, the absorbent article comprises or is a diaper, preferably a smart diaper. Advantageously, such a smart diaper may especially be a diaper comprising at least one sensor and/or sensing means. Further advantageously, additionally or alternatively, such a smart diaper may comprise or be an absorbent article comprising at least one sensor and/or sensing means and/or sensing elements, preferably active and/or passive sensing elements.

According to a second preferred implementation form of the first aspect of the invention, the device identifier is visibly attached to the identification device. In addition to this or as an alternative, the device identifier comprises at least one of a serial number, especially a unique serial number, a label, a quick-response code, a near-field communication tag, especially a passive near-field communication tag, an electronic serial number, preferably an electronic serial number embedded into the respective hardware, or any combination thereof. Advantageously, for instance, production can be standardized and costs can be reduced.

According to a further preferred implementation form of the first aspect of the invention, the personal identifier comprises at least one of an identification number associated to the wearer, the name of the wearer, the initials of the wearer, the location of residence of the wearer, any other kind of personal information with respect to the wearer, or any combination thereof. Advantageously, for example, information can easily be interpreted especially be a caregiver of the wearer.

According to a further preferred implementation form of the first aspect of the invention, the communication between the communication unit and the nearby back-end unit is based on short-distance communication, preferably Near-Field Communication, Bluetooth, especially Bluetooth low energy, ZigBee, or any other kind of short-distance communication technology. Advantageously, for instance, especially due to a standardized communication, production costs can further be reduced.

According to a further preferred implementation form of the first aspect of the invention, the communication unit is configured to communicate with a remote back-end unit. In addition to this or as an alternative, the communication unit is configured to send remote information to the remote back-end unit. In this context, the remote information comprises at least one of the device identifier, a status of the absorbent article, preferably a status of the wetness and/or exudates inside the absorbent article, a status of the identification device, preferably a battery level of the identification device, a status of the communication unit, preferably a status of the connection quality with respect to the communication between the communication unit and the nearby back-end unit and/or the remote back-end unit, a status of the pairing unit, preferably an indication if the personal identifier has successfully been linked to the device identifier, or any combination thereof. Advantageously, for example, diapers and/or wearer can remotely be monitored, which saves time.

According to a further preferred implementation form of the first aspect of the invention, the communication between the communication unit and the remote back-end unit is based on wireless local area network, Bluetooth, especially Bluetooth low energy, ZigBee, or any other kind of wireless communication technology. Advantageously, for instance, communication standardization helps to further reduce production costs.

According to a further preferred implementation form of the first aspect of the invention, the communication unit is configured to receive data, preferably data being independent from the wearer, more preferably configuration data with respect to the identification device, most preferably a firmware update with respect to the identification device, from the remote back-end unit. Advantageously, for example, the identification device can remotely be updated, which ensures a high flexibility and efficiency.

According to a further preferred implementation form of the first aspect of the invention, the communication unit is configured to use two separate communication channels comprising one channel for the communication with the nearby back-end unit and one channel for the communication with the remote back-end unit. Advantageously, for instance, it can efficiently be complied with data protection restrictions.

Further advantageously and preferably, the above-mentioned first communication means may be used for the communication with the nearby back-end unit. In addition to this or as an alternative, the above-mentioned second communication means may be used for the communication with the remote back-end unit.

According to a further preferred implementation form of the first aspect of the invention, the communication unit is configured to send nearby information to the nearby back-end unit. In this context, the nearby information comprises at least one of the device identifier, the personal identifier, a status of the identification device, preferably a battery level of the identification device, a status of the communication unit, preferably a status of the connection quality with respect to the communication between the communication unit and the nearby back-end unit and/or, especially in the case of the presence of the remote back-end unit, the remote back-end unit, a status of the pairing unit, preferably an indication if the personal identifier has successfully been linked to the device identifier, or any combination thereof. Advantageously, for example, data security with respect to the communication can further be increased.

According to a further preferred implementation form of the first aspect of the invention, the identification device comprises a feedback unit. In this context, the feedback unit is configured to provide a feedback for a user, preferably a caregiver of the wearer, and/or the wearer with respect to at least one of the device identifier, the personal identifier, a status of the identification device, preferably a battery level of the identification device, a status of the communication unit, preferably a status of the connection quality with respect to the communication between the communication unit and the nearby back-end unit and/or, especially in the case of the presence of the remote back-end unit, the remote back-end unit, a status of the pairing unit, preferably an indication if the personal identifier has successfully been linked to the device identifier, or any combination thereof. Advantageously, for instance, said feedback allows for a particularly efficient usage of the identification device.

According to a further preferred implementation form of the first aspect of the invention, the feedback comprises at least one of an optical feedback, an acoustical feedback, a haptic feedback, or any combination thereof. In addition to this or as an alternative, the feedback unit comprises at least one of a screen or a display or a light, preferably a signal light, a speaker or a beeper, a vibration unit or a vibration motor, or any combination thereof. Further additionally or further alternatively, the feedback unit is configured to provide instructions for a user, preferably a caregiver of the wearer, and/or the wearer especially with respect to configuration and/or usage of the identification device. In further addition to this or as a further alternative, in the case of detachment of the identification device from the absorbent article, the feedback unit is configured to ask and/or warn a user, preferably a caregiver of the wearer, and/or the wearer if said detachment was intended. Advantageously, for example, the complexity of the feedback can be reduced, which leads to a further increased efficiency.

According to a second aspect of the invention, an identification system for an absorbent article of a wearer is provided. The identification system comprises an identification device, especially an identification device according to the first aspect of the invention and/or any of its preferred implementation forms, comprising a device identifier, a communication unit, and a pairing unit connected to the communication unit, and a nearby back-end unit. In this context, the communication unit is configured to receive a personal identifier with respect to the wearer from the nearby back-end unit and to provide the personal identifier for the pairing unit. In addition to this, the pairing unit is configured to link the personal identifier to the device identifier. Advantageously, both a particularly high flexibility and a particularly high efficiency are ensured.

Further advantageously and preferably, the identification system may comprise a remote back-end unit. In this context, the communication unit may be configured to communicate with the remote back-end unit. In addition to this or as an alternative, the communication unit may be configured to use two separate communication channels comprising one channel for the communication with the nearby back-end unit and one channel for the communication with the remote back-end unit.

As a further advantage, the nearby back-end unit may comprise or be a user equipment of a user, especially a caregiver of the wearer, and/or the wearer, preferably a mobile device or a mobile phone. In addition to this or as an alternative, the remote back-end unit may comprise or be a server, preferably a cloud server.

According to a third aspect of the invention, a usage method for using an identification device for an absorbent article of a wearer is provided. The usage method comprises the steps of pairing an identification device according to the first aspect of the invention and/or any of its preferred implementation forms with a personal identifier with respect to the wearer, and attaching the identification device to the absorbent article of the wearer. Advantageously, both a particularly high flexibility and a particularly high efficiency are ensured.

According to a fourth aspect of the invention, an array of identification devices for absorbent articles of wearers is provided. The array comprises at least two identification devices according to the first aspect of the invention and/or any of its preferred implementation forms. In this context, each of a part of the at least two identification devices is paired with a personal identifier with respect to a corresponding wearer and attached to the respective absorbent article. In addition to this, each of a part of the at least two identification devices is charging or ready for use. Advantageously, both a particularly high flexibility and a particularly high efficiency are ensured.

Exemplary embodiments of the invention are now further explained with respect to the drawings by way of example only, and not for limitation. In the drawings:
- Fig. 1: shows an exemplary embodiment of the inventive identification device and system;
- Fig. 2: shows a flow chart of an exemplary embodiment of the inventive usage method;
- Fig. 3: shows a flow chart of a further exemplary embodiment of the inventive usage method;
- Fig. 4: shows a flow chart of a further exemplary embodiment of the inventive usage method;
- Fig. 5: shows different exemplary means for device identification;
- Fig. 6: shows an exemplary embodiment of an inventive feedback unit;
- Fig. 7: shows an exemplary embodiment of the inventive array;
- Fig. 8: shows a further exemplary embodiment of the inventive array;
- Fig. 9: shows a second exemplary embodiment of the inventive system in the context of a general use case;
- Fig. 10: shows the second exemplary embodiment of the inventive system in the context of pairing;
- Fig. 11: shows the corresponding data flow for pairing with respect to Fig. 10;
- Fig. 12: shows the second exemplary embodiment of the inventive system in the context of pairing in an alternative manner;
- Fig. 13: shows the corresponding data flow for pairing with respect to Fig. 12;
- Fig. 14: shows the second exemplary embodiment of the inventive system in the context of monitoring; and
- Fig. 15: shows the corresponding data flow for monitoring with respect to Fig. 14.

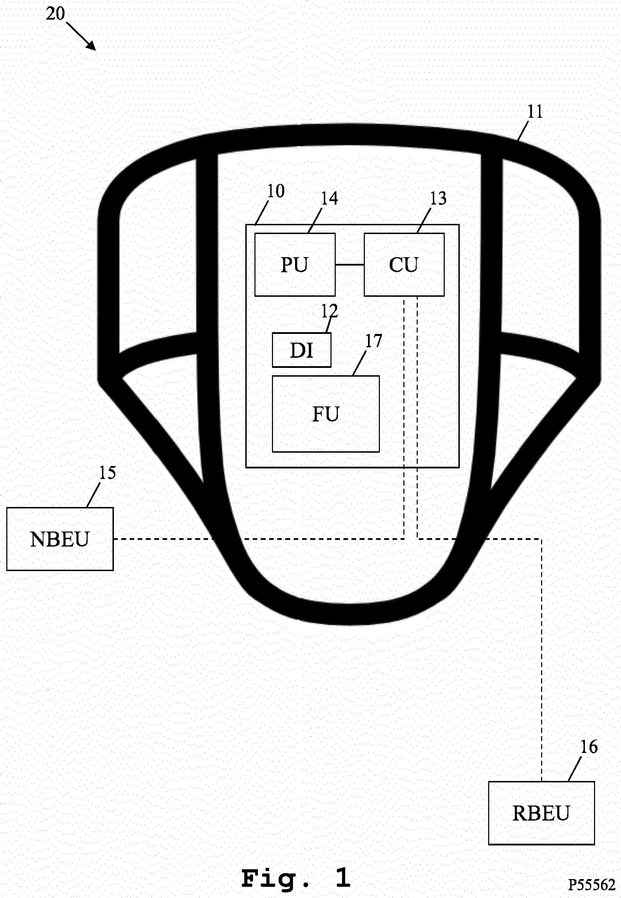

Firstly, an exemplary embodiment of an identification device 10 for an absorbent article, exemplarily a diaper 11, of a wearer and an exemplary embodiment of an identification system 20 for the diaper 11 of the wearer are shown in accordance with Fig. 1. Said identification device 10 comprises a device identifier 12, a communication unit 13, and a pairing unit 14 connected to the communication unit 13. In this context, the communication unit 13 is configured to receive a personal identifier with respect to the wearer from a nearby back-end unit 15 and to provide the personal identifier for the pairing unit 14. In addition to this, the pairing unit 14 is configured to link the personal identifier to the device identifier 12.

It is noted that it might be particularly advantageous if the personal identifier is systemically and/or locally linked to the device identifier 12 especially on the side of the identification device 10. In addition to this or as an alternative, the identification device 10 is attached to the diaper 11. Further additionally or further alternatively, the diaper 11 may especially be a smart diaper. Advantageously, the device may preferably be configured to sense the presence of wetness and/or exudates within such a smart diaper to notify the wearer of the diaper and/or a caregiver of the wearer, when the diaper is wet and/or contains exudates and/or the diaper has to be changed.

With respect to the device identifier 12, it is noted that the device identifier 12 may visibly be attached to the identification device 10. In addition to this or as an alternative, the device identifier 12 may comprise at least one of a serial number, especially a unique serial number, a label, a quick-response (QR) code, a near-field communication (NFC) tag, especially a passive near-field communication tag, an electronic serial number, preferably an electronic serial number embedded into the respective hardware, or any combination thereof.

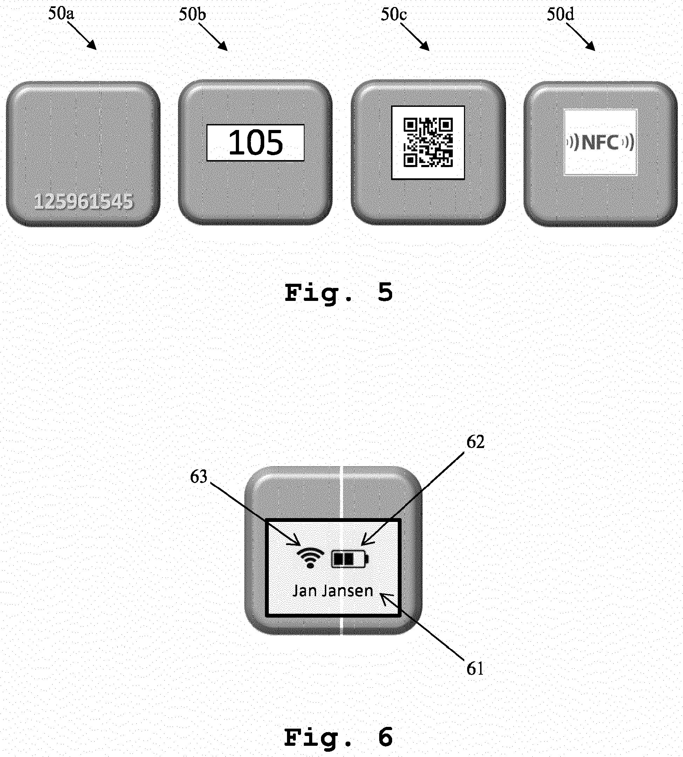

Such different exemplary means for device identification are illustrated by Fig. 5. The first means 50a comprises the above-mentioned unique serial number. It might be particularly advantageous if said unique serial number is visible on the outer shell of the device, made for example with laser engraving upon manufacturing of the device. This serial number can also be taken up within the hardware, which would make it an electronic serial number. The identifier may be assigned to a certain person's profile and to determine if the device is active and to whom the device is paired. For performing a new pairing or unpairing action with that device, one can look up the serial number through software. Advantageously, a lowest cost of components can be ensured.

The second means 50b comprises the above-mentioned label (exemplarily "105"), which can be attached to the device, containing limited personal information such as a person's initials or a person's room number within an institution. This can be done with durable stickers. Additionally, an engraved serial number can be on a device as already mentioned above. To determine if the device is active and whom a device is paired to, and for performing a new pairing or unpairing action with that device, one can look up the serial number through software. Advantageously, such a label is easier for a caregiver to interpret than a random serial number.

Furthermore, the third means 50c comprises the above-mentioned QR code which can be scanned with e.g. a smartphone's camera to receive information about connectivity and pairing status of the device and for performing a new pairing or unpairing action with that device. Advantageously, production of devices can be standardized (vs. e.g. customized room number list). Further advantageously, devices can still be assigned to any resident/any room number.

The fourth means 50d comprises the above-mentioned passive NFC tag, which can be scanned with e.g. a smartphone's NFC reader to receive information about connectivity and pairing status of the device and for performing a new pairing or unpairing action with that device. Advantageously, production of devices can be standardized (vs. e.g. customized room number list). As a further advantage, devices can still be assigned to any resident/any room number. It is noted that the above-mentioned means for device identification 50a, 50b, 50c, 50d are especially passive means.

Alternatively, active means can be foreseen, for which additional components can be built in device, that allow direct communication between device and e.g. a smartphone; NFC technology would be an example technology that can be used, or Bluetooth or other near field communication technologies. A smartphone can be brought into proximity to the device to receive information about connectivity and pairing status, and for performing a new pairing or unpairing action with that device. This requires interaction with a smartphone for performing these actions, but upon getting used to such a system this might bring along the most user friendly way to perform such a pairing action.

Such active means are illustrated by Fig. 6. In other words, Fig. 6 shows an exemplary embodiment of an inventive feedback unit 17, which will be explained in the context of Fig. 1 below. With respect to said active means according to Fig. 6, the system or device, respectively, can be made particularly user friendly, through integration with visual feedback components that are integrated within the device so that it can directly display its pairing status. It is for example to made use of LED (light emitting diode) lights that visualize a different color depending on the pairing status, or haptic feedback that confirms the pairing status, or an integrated display that shows the current pairing status.

The system can be made particularly user friendly, through integration with a screen or display within the device that can directly display its pairing status, along with information about which person it is currently paired to. This requires getting personal information to the identification device, e.g. on the screen, which might not be allowable over the same wireless communication protocol as what the device is normally sending information with, due to data protection restrictions.

Therefore, the scenario where any additional component is used that enables e.g. NFC or Bluetooth 2-way-communication, brings the additional advantage of allowing to send personal information (such as the personal identifier, exemplarily in the form of the person's name 61: "Jan Jansen") to a device's local memory, so that it can be visualized on the screen. If this device is detached from the diaper and found somewhere, it can be immediately clear to whom it is currently paired from personal info on display. Advantageously, there is no need to interpret the respective device identifier.

The usage of a screen can bring additional user friendly advantages, such as displaying the current battery status 62 or the connectivity status 63 within the wireless network.

Again, with respect to Fig. 1, the personal identifier may comprise at least one of the name of the wearer as already mentioned with respect to Fig. 6 (reference sign 61), an identification number associated to the wearer, the initials of the wearer, the location of residence of the wearer, any other kind of personal information with respect to the wearer, or any combination thereof.

Furthermore, the communication between the communication unit 13 and the nearby back-end unit 15 may be based on short-distance communication such as Near-Field Communication, Bluetooth, especially Bluetooth low energy, ZigBee, or any other kind of short-distance communication technology. Moreover, the communication unit 13 is configured to communicate with a remote back-end unit 16. In addition to this or as an alternative, the communication unit 13 is configured to send remote information to the remote back-end unit 16.

In this context, the remote information may especially comprise at least one of the device identifier 12, a status of the diaper, preferably a status of the wetness and/or exudates inside the diaper 11, a status of the identification device 10, preferably a battery level of the identification device 10, a status of the communication unit 13, preferably a status of the connection quality with respect to the communication between the communication unit 13 and the nearby back-end unit 15 and/or the remote back-end unit 16, a status of the pairing unit 14, preferably an indication if the personal identifier has successfully been linked to the device identifier 12, or any combination thereof.

Furthermore, the communication between the communication unit 13 and the remote back-end unit 16 may be based on wireless local area network, Sigfox, LoRa, Bluetooth, especially Bluetooth low energy, ZigBee, or any other kind of wireless communication technology. In this context, the communication unit 13 may especially be configured to receive data, preferably data being independent from the wearer, more preferably configuration data with respect to the identification device 10, most preferably a firmware update with respect to the identification device 10, from the remote back-end unit 16. Moreover, the communication unit 13 may be configured to use two separate communication channels comprising one channel for the communication with the nearby back-end unit 15 and one channel for the communication with the remote back-end unit 16.

It is further noted that the communication unit 13 may especially be configured to send nearby information to the nearby back-end unit 15. In this context, the nearby information comprises at least one of the device identifier 12, the personal identifier, a status of the identification device 10, preferably a battery level of the identification device 10, a status of the communication unit 13, preferably a status of the connection quality with respect to the communication between the communication unit 13 and the nearby back-end unit 15 and/or the remote back-end unit 16, a status of the pairing unit 14, preferably an indication if the personal identifier has successfully been linked to the device identifier 12, or any combination thereof.

As it can further be seen from Fig. 1 and as already mentioned above, the identification device 10 comprises a feedback unit 17. In this context, the feedback unit 17 is configured to provide a feedback for a user, preferably a caregiver of the wearer, and/or the wearer with respect to at least one of the device identifier 12, the personal identifier, a status of the identification device 10, preferably a battery level of the identification device 10, a status of the communication unit 13, preferably a status of the connection quality with respect to the communication between the communication unit 13 and the nearby back-end unit and/or the remote back-end unit 16, a status of the pairing unit 14, preferably an indication if the personal identifier has successfully been linked to the device identifier 12, or any combination thereof.

It might be particularly advantageous if the feedback comprises at least one of an optical feedback, an acoustical feedback, a haptic feedback, or any combination thereof. In addition to this or as an alternative, the feedback unit 17 may comprise at least one of a screen or a display such as the embodiment according to Fig. 6, a speaker or a beeper, a vibration unit or a vibration motor, or any combination thereof.

Further additionally or further alternatively, the feedback unit 17 may be configured to provide instructions for a user, preferably a caregiver of the wearer, and/or the wearer especially with respect to configuration and/or usage of the identification device 10. In further addition to this or as a further alternative, especially in the case of detachment of the identification device 10 from the diaper 11, the feedback unit 17 may be configured to ask and/or warn a user, preferably a caregiver of the wearer, and/or the wearer if said detachment was intended.

As already mentioned above, Fig. 1 also shows an exemplary embodiment 20 of the inventive identification system, which comprises the identification device 10 comprising the device identifier 12, the communication unit 13, and the pairing unit 14 connected to the communication unit 13, and the nearby back-end unit 15. In this context, the communication unit 13 is configured to receive a personal identifier with respect to the wearer from the nearby back-end unit 15 and to provide the personal identifier for the pairing unit 14. In addition to this, the pairing unit 14 is configured to link the personal identifier to the device identifier 12.

Advantageously, the identification system 20 may comprise the remote back-end unit 16. In this context, the communication unit 13 may be configured to communicate with the remote back-end unit 16. In addition to this or as an alternative, the communication unit 13 may be configured to use two separate communication channels comprising one channel for the communication with the nearby back-end unit 15 and one channel for the communication with the remote back-end unit 16. Further advantageously, the nearby back-end unit 15 may comprise or be a user equipment of a user, especially a caregiver of the wearer, and/or the wearer, preferably a mobile device or a mobile phone. In addition to this or as an alternative, the remote back-end unit 16 may comprise or be a server, preferably a cloud server.

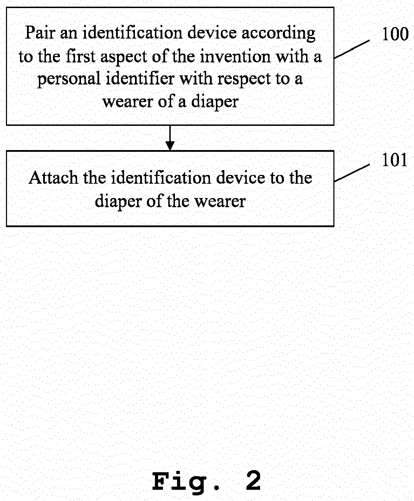

Now, with respect to Fig. 2, a flow chart of an embodiment of the inventive usage method for using an identification device for an absorbent article, exemplarily a diaper, of a wearer is shown. According to a first step 100, the usage method comprises pairing an identification device such as the above-mentioned device 10 with a personal identifier with respect to the wearer. Then, in accordance with a second step 101, the usage method comprises attaching the identification device to the diaper of the wearer.

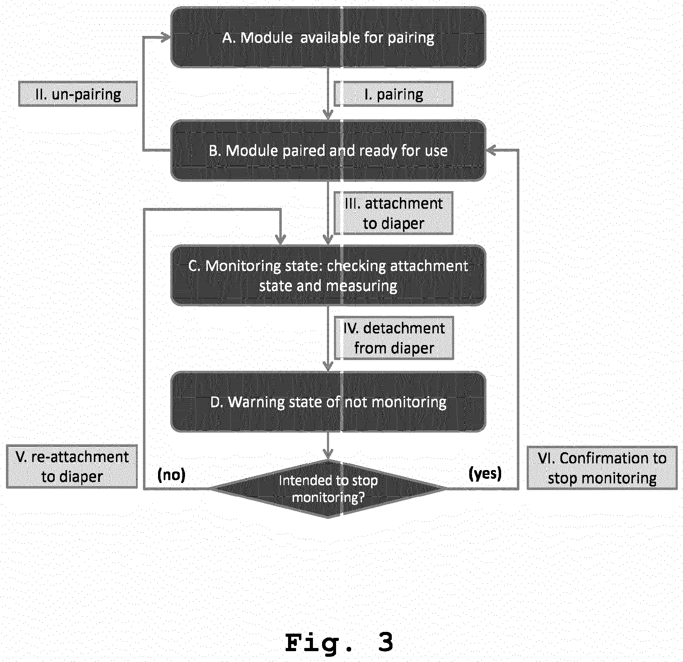

Furthermore, Fig. 3 illustrates a further exemplary embodiment of the inventive usage method. In this context, it is noted that the identification device advantageously comprises a monitoring unit being configured to monitor a state of the diaper and/or of the wearer of the diaper.

In accordance with Fig. 3, any device can typically be activated for use (status A), after which it may be paired to a person's personal identifier (I. Pairing). The step of pairing especially involves creating a link between the device and the person's personal identifier, that allows for assigning data that will be sent by this respective device automatically to that person's profile, and that will add the person's details (preferably, the person's name, initials, location of residence, ...) towards a caregiver upon receiving a notification about the person's status.

After pairing (I.), the respective device is paired and ready for use according to state B. In this context, a step of un-pairing (II.) would lead from state B to the above-mentioned state A.

When a device is paired and ready for use (status B), it can be attached to a diaper (III.) which will automatically be recognized by the identification device and which will activate the monitoring of the device (sending data wirelessly, for updating the person's information and storing it, and for notifying a caregiver about status changes). Accordingly, the respective device is in monitoring state (C.), which especially comprises checking attachment state and performing measurements with respect to the diaper and/or the wearer of the diaper.

Moreover, a detachment from the diaper (IV.) would lead to a warning state (D.) of not monitoring. In the case that it is not intended to stop monitoring, the respective device should be re-attached to the diaper (V.). Further interaction such as the stopping of monitoring (VI.) or a confirmation thereof, respectively, is also enabled. As already mentioned above, unpairing of a device is also possible, and removes the link between the person's personal identifier and the device - making the device available to be paired again to the same person's or to another person's personal identifier.

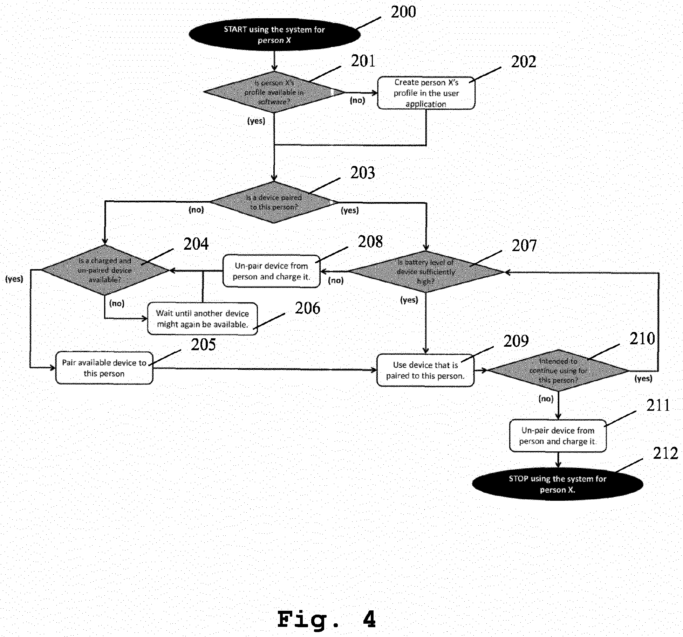

Now, with respect to Fig. 4, a further exemplary embodiment of the inventive usage method is explained, which is especially focused on pairing and un-pairing. In a first step 200, the inventive system or device, respectively, should be used for a certain person. In a second step 201, it is checked if the profile of the certain person is available especially in a respective software. In the case that it is not available, a profile of the certain person is created especially in the corresponding user application according to step 202.

Afterwards, in accordance with step 203, it is checked if a device, especially any device, is paired to the certain person. In the case that no device is already paired to the certain person, it is checked if a charged and un-paired device is available according to step 204. If a device is available, this device is paired to the certain person according to step 205. If no device is available, it is waited until another device might again be available according to step 206.

In the case that a device is already paired to the certain person, it is checked if the respective battery level of the corresponding device is sufficiently high according to step 207. If said battery level is too low, the corresponding device is un-paired from the respective person and charged according to step 208. If said battery level is high enough, the device being paired to this person is used according to step 209.

After said step 209, it is checked if the respective device is intended to continue using for this person according to step 210. If not, the respective device is un-paired from the respective person and charged according to step 211, which leads to step 212 (stop using the system for the certain person). In the other case, it is gone back to the above-mentioned step 207.

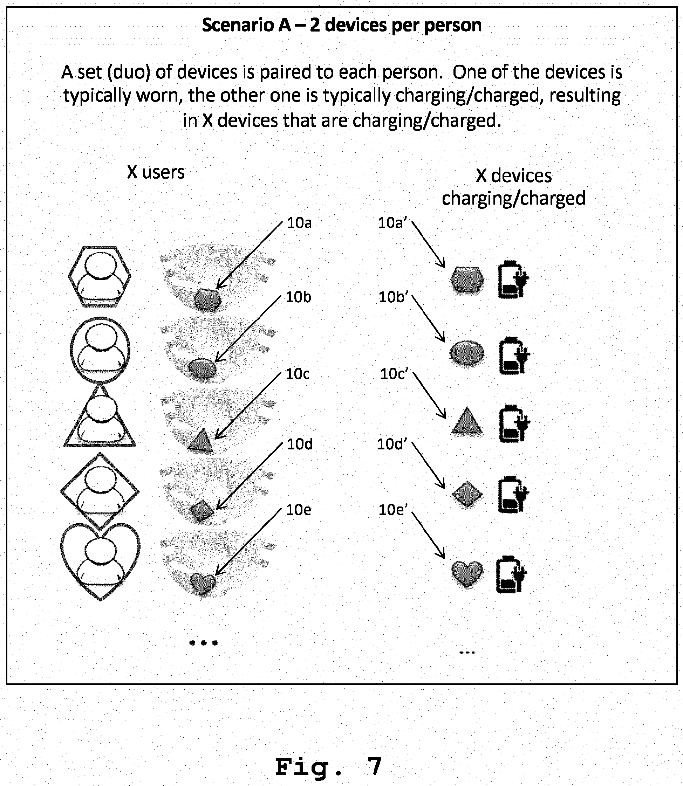

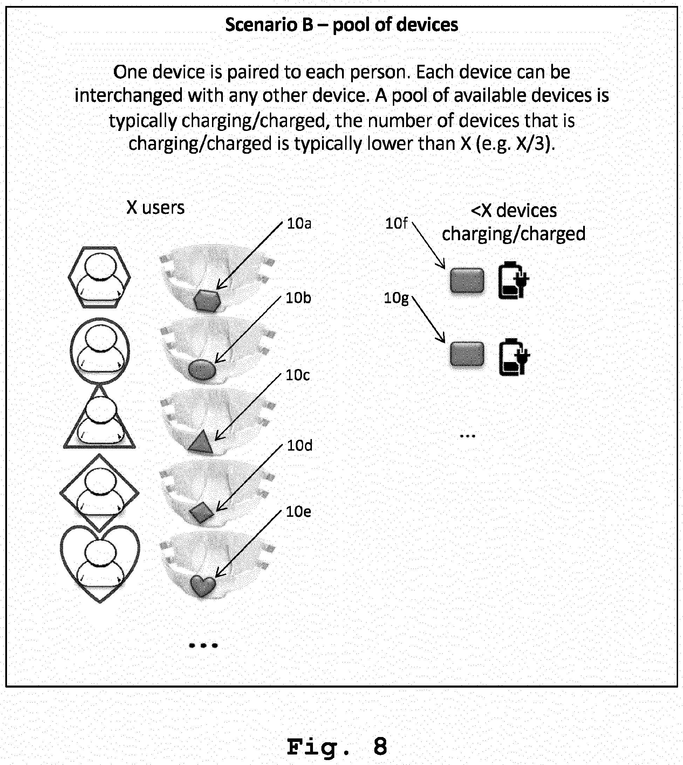

Furthermore, with respect to Fig. 7 and Fig. 8, exemplary embodiments of the inventive array of identification devices for diapers of wearers are shown. Basically, such an inventive array comprises at least two inventive identification devices such as the device 10 according to Fig. 1. In this context, each of a part of the at least two identification devices is paired with a personal identifier with respect to a corresponding wearer and attached to the respective diaper. In addition to this, each of a part of the at least two identification devices is charging or ready for use.

Before said exemplary embodiments according to Fig. 7 and Fig. 8, more detailed background information in the sense of the invention will be given in the following. A smart diaper especially combines an inventive identification device, for instance, in the form of a re-usable clip-on device, with a disposable modified diaper, especially adult diaper, that contains a sensor.

By wearing the combination of the inventive device and a disposable diaper, several measurement and sensor functionalities can be enabled, such as the monitoring of wetness and/or exudates inside the modified adult diaper or the detection of fall incidents. What groups the aimed at functionalities, is that it is the intention to notify a caregiver about the situation (= alerting) through wireless communication from the device towards a caregiver, for example through a software application. It is important that a caregiver can, upon being notified about the situation, immediately identify to which person the notification applies.

This can be achieved through linking a device identifier to a personal identifier (of the person who will be wearing the device), and having this link known within the exemplary software application as already mentioned above.

The typical battery lifetime of such a device will require charging after certain intervals of use time, such as one week. It may be inconvenient that during the charging time of such a device, it would not be possible to monitor the functionalities. An exemplary goal is to use the invention in nursing homes. In such institutions, there will typically be a continuous flow of incoming residents and people who move out (e.g. for hospitalization) and a high rate of people that pass away will be found.

Besides, only a part of the current population within one nursing home may typically make use of the functionalities of this system or device, respectively, whilst others don't suffer from incontinence. Therefore, it is very relevant that devices can be used interchangeably, and therefore such devices would typically be managed and/or owned by the institution and not by the person who is wearing the device. There may be a need for continuous monitoring, for people to whom it is relevant and independently of the charging need for such a device. This thus requires a versatile approach, which leads to the embodiments according to Fig. 7 and Fig. 8.

In accordance with Fig. 7, exemplarily called scenario A, two devices (pairs 10a and 10a', 10b and 10b', 10c and 10c', 10d and 10d', 10e and 10e') can be assigned to each person, to allow for monitoring also during charging time of a first device (by the second spare device), and vice versa. A pairing step from a device to the person's personal identifier would be required once upon starting the use of the functionalities for this person, an un-pairing step would be required once upon ending the use of the functionalities for this person. Accordingly, according to scenario A, if there are X users, 2X devices are required: X devices being in use, and X devices charging or being ready for use.

In accordance with Fig. 8, exemplarily called scenario B, a pool of devices is used, where the pool of charging devices 10f, 10g is not assigned to personal identifiers, but the set of devices 10a, 10b, 10c, 10d, 10e that is currently in use is linked to the personal identifier of the person that is respectively wearing the device. A pairing step from the device to the person's personal identifier would be required upon every start of monitoring with a freshly charged device for this person, potentially combined with an un-pairing step of such a device.

Accordingly, according to scenario B, if there are X users, less than 2X devices are required: X devices being in use, and less than X devices charging or being ready for use, preferably X/2 devices charging or being ready for use, more preferably X/4 devices charging or being ready for use, most preferably X/3 devices charging or being ready for use. As it can be seen, scenario B might be beneficial over scenario A, because it requires a much lower amount of devices to cover the same group of people, in case the time to reach full charge status of a battery is limited (for instance, 2 hours of charging vs. 1 week lifetime of a battery).

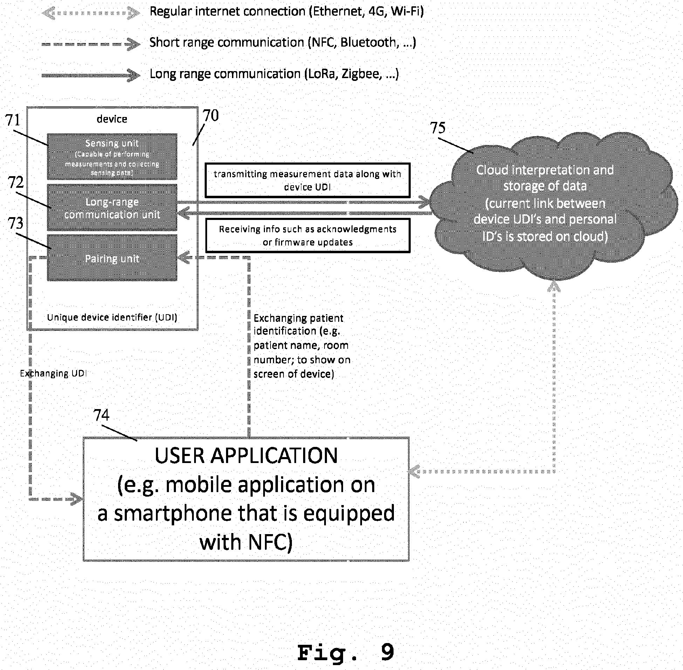

Now, with respect to Fig. 9, in addition or as an alternative to the first exemplary embodiment according to Fig. 1, a second exemplary embodiment of the inventive system is illustrated.

In accordance with Fig. 9, said system comprises an identification device 70, a user application 74, which especially is a kind of the nearby back-end unit, and a cloud 75, preferably a cloud server, which especially is a kind of the remote back-end unit.

In this context, said identification device 70 comprises a sensing unit 71 configured to perform measurements and/or to collect sensing data especially with respect to the corresponding diaper and/or the wearer of the diaper, a communication unit, exemplarily a long-range communication unit 72, and a pairing unit 73.

Moreover, the pairing unit 73 is configured to exchange the respective unique device identifier (UDI) of the identification device 70 with the user application 74 especially via short range communication such as NFC or Bluetooth.

With respect to the user application 74, it is noted that the user application 74 may preferably comprise a mobile application on a smartphone that may especially be equipped with NFC.

Furthermore, the user application 74 is configured to exchange the identification of the corresponding wearer or patient, respectively, with the pairing unit 73 especially via the short range communication. Said identification may preferably comprise the patient's name and/or room number. Additionally or alternatively, said identification may be shown on a screen (not explicitly depicted) of the identification device 70.

Moreover, the user application 74 is configured to exchange data with the cloud 75 especially via an internet connection such as Ethernet, 4G, or Wi-Fi.

The cloud 75 is configured to interpret and/or store data. In this context, the respective current link between identification device UDIs and personal IDs or wearer identifications, respectively, may preferably be stored on the cloud 75.

With respect to the communication unit 72, it is noted that the communication unit 72 is configured to transmit the respective measurement data especially along with the UDI to the cloud 75 preferably via a long range communication such as LoRa (long range) or ZigBee. Additionally or alternatively, the communication unit 72 is configured to receive information such as acknowledgements and/or firmware updates from the cloud 75 especially via the long range communication.

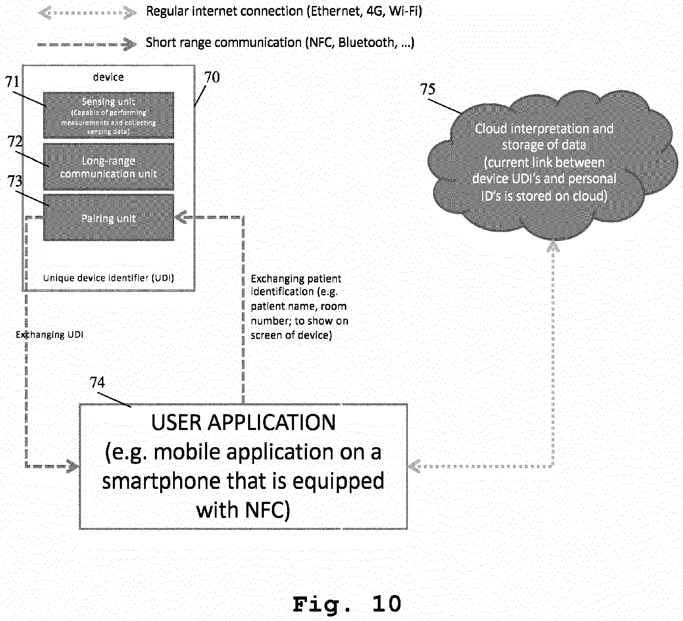

In accordance with Fig. 10, the second embodiment of the inventive system is shown in the context of pairing. As it can be seen, in this exemplary use case, the above-mentioned long range communication is not necessary.

Advantageously, according to Fig. 10, a particularly data safety can be ensured because it is possible to bring personal information to the device 70 through a separate communication channel.

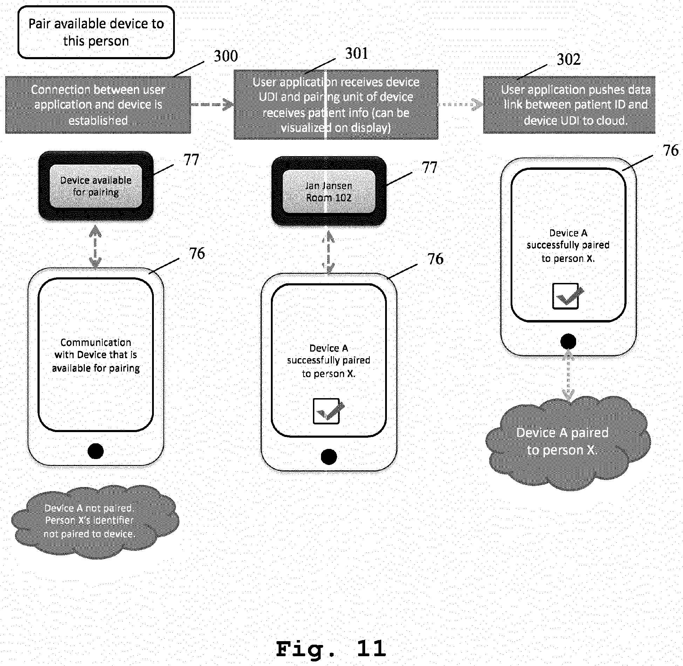

The corresponding data flow for pairing with respect to Fig. 10, is illustrated by Fig. 11. In this context, in a first step 300, the respective connection between the user application and the device is established. Accordingly, an exemplary screen 77 of the device may show a message that the respective device is available for pairing. Furthermore, the user application, exemplarily the smartphone 76 may show a message that there is performed a communication with the device being available for pairing. In accordance with said step 300, the device, exemplarily device A, is not paired and person X's identifier is not paired to the device A, which is exemplarily stored on the respective cloud.

Moreover, in a second step 301, the user application receives the respective UDI and the pairing unit of the respective device receives the corresponding information of the wearer of the diaper, exemplarily the corresponding patient information. This can also be visualized by the display or screen 77, respectively, which shows the name, exemplarily "Jan Jansen", of the wearer, and the room number, exemplarily 102, of the wearer. Further exemplarily, the smartphone 76 shows a message that the device A has successfully been paired to said person X.

In accordance with a third step 302, the user application pushes the respective data link between the patient ID and the UDI to the respective cloud. Accordingly, the cloud stores that device A is paired to person X.

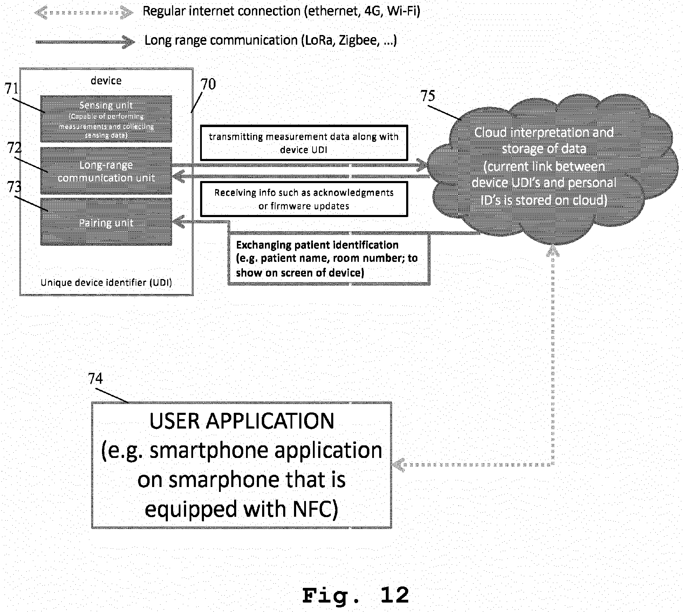

Now, with respect to Fig. 12, an alternative of pairing is illustrated, which especially differs from Fig. 10 in that the patient identification such as the patient's name or the patient's room number is exchanged via the long range communication instead of the short range communication. It is noted that this alternative of pairing is a non-preferred one because of reduced data safety due to the usage of the same communication channel.

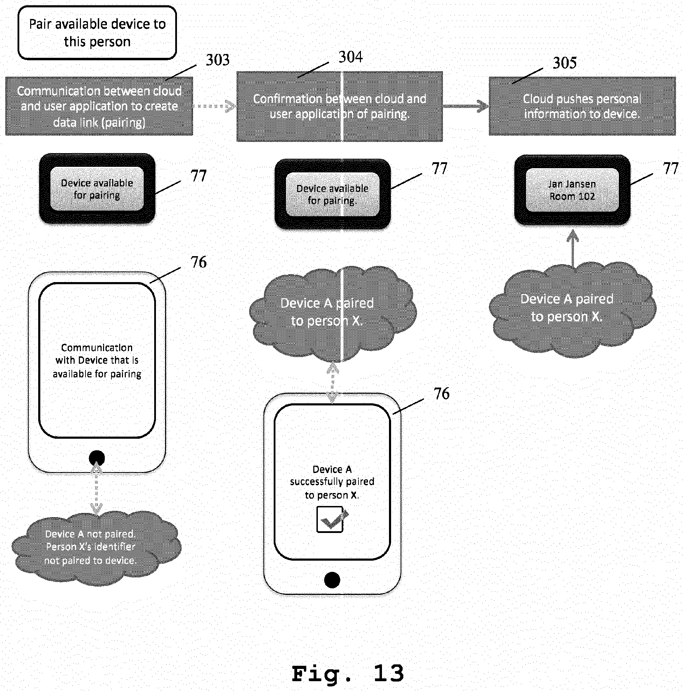

In addition to this, Fig. 13 shows the corresponding data flow for pairing with respect to said non-preferred alternative. In this context, in a first step 303, there is performed a communication between the respective cloud and the respective user application to create the corresponding data link (pairing). Exemplarily, the screen 77 may show a message that the respective device is available for pairing, whereas the smartphone 76 may show a message that there is a communication with the respective device being available for pairing. In addition to this, the smartphone 76 exchanges the information that the device A is not paired and the person X's identifier is not paired to the device with the respective cloud especially via the internet connection.

Furthermore, in a second step 304, pairing is confirmed between the cloud and the user application. Exemplarily, the message shown on the screen 77 does not change especially with respect to the previous step 303. Additionally, the smartphone 76 exemplarily exchanges the message that the device A is paired to person X with the cloud especially via the internet connection.

According to a third step 305, the cloud pushes the respective personal information to the device especially via the long range communication. As it can be seen, the screen 77 then shows the patient's name and room number.

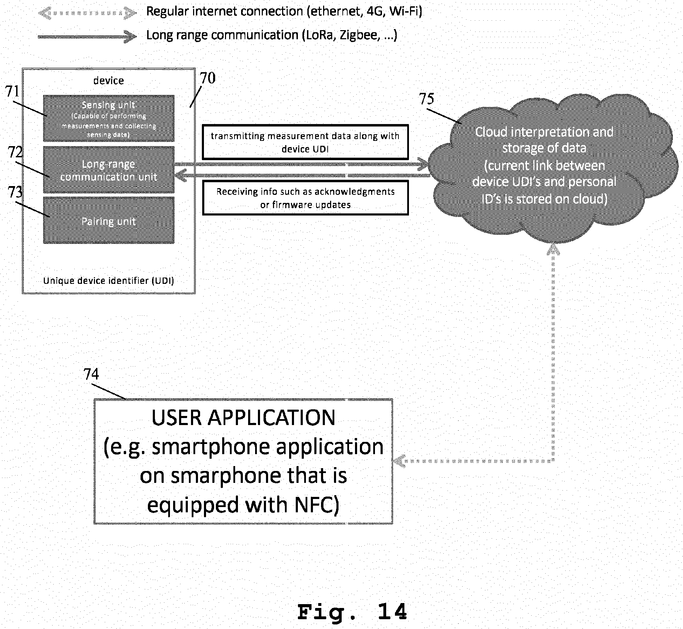

Moreover, in the context of monitoring, Fig. 14 illustrates that no patient identification has to be exchanged. In addition to this, a short range communication is not necessary.

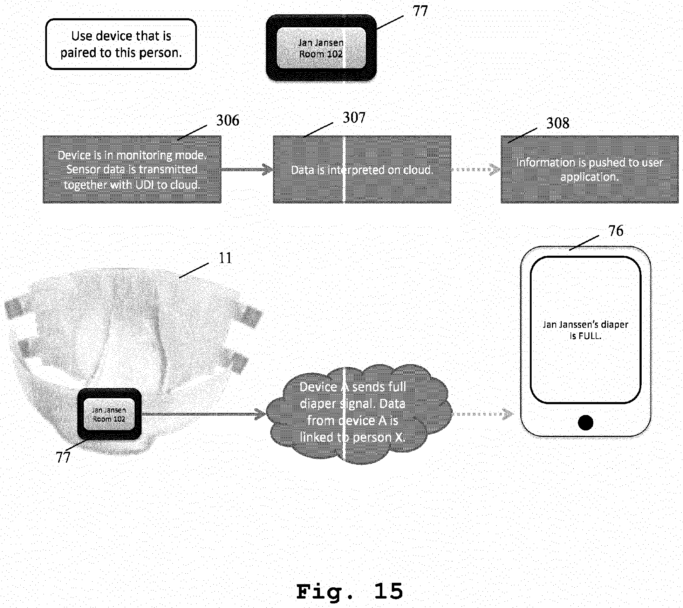

Finally, Fig. 15 shows the corresponding data flow for monitoring with respect to Fig. 14. According to a first step 306, the respective device is in monitoring state. Additionally, sensor data is transmitted together with the respective UDI to the cloud. As it can be seen, the screen 77 of the device attached to the diaper 11 shows the patient's name and room number.

In accordance with a second step 307, the respective data is interpreted on the cloud. It is noted that the device A may send the corresponding full diaper signal and the data from the device A is linked to person X. Said full diaper signal may especially be sent via the long range communication.

Furthermore, according to a third step 308, the respective information is pushed to the user application especially via the internet connection. Exemplarily, the smartphone 76 may show a message that the respective diaper (Jan Jansen's one) is full.

With respect to the above-mentioned visualization of the respective patient information on the display or the screen 77, respectively, it is noted that said visualization may be particularly advantageous if the respective device is detached from the corresponding diaper and found somewhere. In this case, it is immediately clear to whom it is currently paired. Further advantageously, there is no need to interpret the respective device UDI.

While various embodiments of the present invention have been described above, it should be understood that they have been presented by way of example only, and not limitation. Numerous changes to the disclosed embodiments can be made in accordance with the disclosure herein without departing from the scope of the invention defined by the claims. Thus, the breadth and scope of the present invention should not be limited by any of the above described embodiments. Rather, the scope of the invention should be defined in accordance with the following claims and their equivalents.

Although the invention has been illustrated and described with respect to one or more implementations, equivalent alterations and modifications will occur to others skilled in the art upon the reading and understanding of this specification and the annexed drawings. In addition, while a particular feature of the invention may have been disclosed with respect to only one of several implementations, such feature may be combined with one or more other features of the other implementations as may be desired and advantageous for any given or particular application.

## Claims

1. An identification device (10) for an absorbent article of a wearer, the identification device (10) comprising:
a device identifier (12),
a communication unit (13), and
a pairing unit (14) connected to the communication unit (13),
wherein the communication unit (13) is configured to receive a personal identifier with respect to the wearer from a nearby back-end unit (15) and to provide the personal identifier for the pairing unit (14), and
wherein the pairing unit (14) is configured to link the personal identifier to the device identifier (12).

2. The identification device (10) according to claim 1, wherein the personal identifier is linked to the device identifier (12) especially on the side of the identification device (10),
wherein the identification device (10) is attached to the absorbent article, and
wherein the absorbent article comprises or is a diaper (11), and
wherein the identification device (10) comprises a monitoring unit being configured to monitor at least one state of the absorbent article, preferably wetness and/or exudates inside the absorbent article, and/or at least one state with respect to the wearer.

3. The identification device (10) according to claim 1 or 2,
wherein the device identifier (12) is visibly attached to the identification device (10), and/or
wherein the device identifier (12) comprises at least one of a serial number, a label (50b), a quick-response code (50c), a near-field communication tag, an electronic serial number, or any combination thereof.

4. The identification device (10) according to any of the claims 1 to 3,
wherein the personal identifier comprises at least one of an identification number associated to the wearer, the name of the wearer, the initials of the wearer, the location of residence of the wearer, any other kind of personal information with respect to the wearer, or any combination thereof.

5. The identification device (10) according to any of the claims 1 to 4,
wherein the communication between the communication unit (13) and the nearby back-end unit (15) is based on short-distance communication and
wherein the communication unit (13) comprises first communication means for the short-distance communication and second communication means for long-distance communication.

6. The identification device (10) according to any of the claims 1 to 5,
wherein the communication unit (13) is configured to communicate with a remote back-end unit (16), and
wherein the communication unit (13) is configured to send remote information to the remote back-end unit (16),
wherein the remote information comprises at least one of the device identifier (12), a status of the absorbent article, a status of the wetness and exudates inside the absorbent article, a status of the identification device (10), a status of the communication unit (13), a status of the connection quality with respect to the communication between the communication unit (13) and the nearby back-end unit (15) and/or the remote back-end unit (16), a status of the pairing unit (14), an indication if the personal identifier has successfully been linked to the device identifier (12), or any combination thereof.

7. The identification device (10) according to claim 6, wherein the communication between the communication unit (13) and the remote back-end unit (16) is based on wireless local area network, Bluetooth, ZigBee, or any other kind of wireless communication technology.

8. The identification device (10) according to claim 6 or 7,
wherein the communication unit (13) is configured to receive data, said data being independent from the wearer, more preferably configuration data with respect to the identification device (10), most preferably a firmware update with respect to the identification device (10), from the remote back-end unit (16).

9. The identification device (10) according to any of the claims 6 to 8,
wherein the communication unit (13) is configured to use two separate communication channels comprising one channel for the communication with the nearby back-end unit (15) and one channel for the communication with the remote back-end unit (16).

10. The identification device (10) according to any of the claims 1 to 9,
wherein the communication unit (13) is configured to send nearby information to the nearby back-end unit (15),
wherein the nearby information comprises at least one of the device identifier (12), the personal identifier, a status of the identification device (10), a status of the communication unit (13), a status of the connection quality with respect to the communication between the communication unit (13) and the nearby back-end unit (15) and/or, in the case of claims 6 to 9 concerning the remote back-end unit (16), a status of the pairing unit (14), an indication if the personal identifier has successfully been linked to the device identifier (12), or any combination thereof.

11. The identification device (10) according to any of the claims 1 to 10,
wherein the identification device (10) comprises a feedback unit (17),
wherein the feedback unit (17) is configured to provide a feedback for a user, and the wearer with respect to at least one of the device identifier (12), the personal identifier, a status of the identification device (10), a status of the communication unit (13), a status of the connection quality with respect to the communication between the communication unit (13) and the nearby back-end unit (15) and, in the case of claims 6 to 9 concerning the remote back-end unit (16), a status of the pairing unit (14), an indication if the personal identifier has successfully been linked to the device identifier (12), or any combination thereof.

12. The identification device (10) according to claim 11, wherein the feedback comprises at least one of an optical feedback, an acoustical feedback, a haptic feedback, or any combination thereof, and
wherein the feedback unit (17) comprises at least one of a screen or a display or a light, a speaker or a beeper, a vibration unit or a vibration motor, or any combination thereof, and
wherein the feedback unit (17) is configured to provide instructions for a user, and/or the wearer especially with respect to configuration and/or usage of the identification device (10), and
wherein, in the case of detachment of the identification device (10) from the absorbent article, the feedback unit (17) is configured to ask and/or warn a user, and/or the wearer if said detachment was intended.

13. An identification system (20) for an absorbent article of a wearer, the identification system (20) comprising:
an identification device (10) comprising a device identifier (12), a communication unit (13), and a pairing unit (14) connected to the communication unit (13), and
a nearby back-end unit (15),
wherein the communication unit (13) is configured to receive a personal identifier with respect to the wearer from the nearby back-end unit (15) and to provide the personal identifier for the pairing unit (14), and
wherein the pairing unit (14) is configured to link the personal identifier to the device identifier (12).

14. A usage method for using an identification device (10) for an absorbent article of a wearer, the usage method comprising the steps of:
pairing an identification device (10) according to any of the claims 1 to 12 with a personal identifier with respect to the wearer, and
attaching the identification device (10) to the absorbent article of the wearer.

15. An array of identification devices for absorbent articles of wearers, the array comprising:
at least two identification devices according to any of the claims 1 to 12,
wherein each of a part of the at least two identification devices is paired with a personal identifier with respect to a corresponding wearer and attached to the respective absorbent article, and
wherein each of a part of the at least two identification devices is either charging or ready for use.

## Patentansprüche

1. Identifizierungsvorrichtung (10) für einen Absorptionsartikel eines Trägers, die Identifizierungsvorrichtung (10) umfassend:
eine Vorrichtungskennung (12),
eine Kommunikationseinheit (13), und
eine Paarungseinheit (14), die mit der Kommunikationseinheit (13) verbunden ist,
wobei die Kommunikationseinheit (13) konfiguriert ist, um eine persönliche Kennung in Bezug auf den Träger von einer nahegelegenen Backend-Einheit (15) zu empfangen und um die persönliche Kennung für die Paarungseinheit (14) bereitzustellen, und
wobei die Paarungseinheit (14) konfiguriert ist, um die persönliche Kennung mit der Vorrichtungskennung (12) zu verknüpfen.

2. Identifizierungsvorrichtung (10) nach Anspruch 1,
wobei die persönliche Kennung mit der Vorrichtungskennung (12) verknüpft ist, insbesondere auf der Seite der Identifizierungsvorrichtung (10),
wobei die Identifizierungsvorrichtung (10) an dem Absorptionsartikel befestigt ist, und
wobei der Absorptionsartikel eine Windel (11) umfasst oder ist, und
wobei die Identifizierungsvorrichtung (10) eine Überwachungseinheit umfasst, die konfiguriert ist, um mindestens einen Zustand des Absorptionsartikels, vorzugsweise Feuchtigkeit und/oder Exsudate im Inneren des Absorptionsartikels, und/oder mindestens einen Zustand in Bezug auf den Träger zu überwachen.

3. Identifizierungsvorrichtung (10) nach Anspruch 1 oder 2,
wobei die Vorrichtungskennung (12) sichtbar an der Identifizierungsvorrichtung (10) befestigt ist, und/oder
wobei die Vorrichtungskennung (12) mindestens eines von einer Seriennummer, einem Etikett (50b), einem Quick-Response-Code (50c), einem Nahfeldkommunikations-Tag, einer elektronischen Seriennummer oder eine beliebige Kombination davon umfasst.

4. Identifizierungsvorrichtung (10) nach einem der Ansprüche 1 bis 3,
wobei die persönliche Kennung mindestens eines von einer Identifizierungsnummer, die dem Träger zugeordnet ist, dem Namen des Trägers, den Initialen des Trägers, dem Wohnort des Trägers, einer beliebigen anderen Art von persönlichen Informationen in Bezug auf den Träger oder eine beliebige Kombination davon umfasst.

5. Identifizierungsvorrichtung (10) nach einem der Ansprüche 1 bis 4,
wobei die Kommunikation zwischen der Kommunikationseinheit (13) und der nahegelegenen Backend-Einheit (15) auf Nahbereichskommunikation basiert, und
wobei die Kommunikationseinheit (13) erste Kommunikationsmittel für die Nahbereichskommunikation und zweite Kommunikationsmittel für die Fernkommunikation umfasst.

6. Identifizierungsvorrichtung (10) nach einem der Ansprüche 1 bis 5,
wobei die Kommunikationseinheit (13) konfiguriert ist, um mit einer entfernten Backend-Einheit (16) zu kommunizieren, und
wobei die Kommunikationseinheit (13) konfiguriert ist, um Ferninformationen an die entfernte Backend-Einheit (16) zu senden,
wobei die Ferninformationen mindestens eines von der Vorrichtungskennung (12), einem Status des Absorptionsartikels, einem Status der Feuchtigkeit und Exsudate im Inneren des Absorptionsartikels, einem Status der Identifizierungsvorrichtung (10), einem Status der Kommunikationseinheit (13), einem Status der Verbindungsqualität in Bezug auf die Kommunikation zwischen der Kommunikationseinheit (13) und der nahegelegenen Backend-Einheit (15) und/oder der entfernten Backend-Einheit (16), einem Status der Paarungseinheit (14), einer Angabe, ob die persönliche Kennung erfolgreich mit der Vorrichtungskennung (12) verknüpft wurde, oder eine beliebige Kombination davon umfasst.

7. Identifizierungsvorrichtung (10) nach Anspruch 6,
wobei die Kommunikation zwischen der Kommunikationseinheit (13) und der entfernten Backend-Einheit (16) auf einem drahtlosen lokalen Netzwerk, Bluetooth, ZigBee oder einer beliebigen anderen Art von Drahtloskommunikationstechnologie basiert.

8. Identifizierungsvorrichtung (10) nach Anspruch 6 oder 7,
wobei die Kommunikationseinheit (13) konfiguriert ist, um Daten, wobei die Daten unabhängig von dem Träger sind, mehr bevorzugt Konfigurationsdaten in Bezug auf die Identifizierungsvorrichtung (10), am meisten bevorzugt ein Firmware-Update in Bezug auf die Identifizierungsvorrichtung (10), von der entfernten Backend-Einheit (16) zu empfangen.

9. Identifizierungsvorrichtung (10) nach einem der Ansprüche 6 bis 8,
wobei die Kommunikationseinheit (13) konfiguriert ist, um zwei separate Kommunikationskanäle zu verwenden, umfassend einen Kanal für die Kommunikation mit der nahegelegenen Backend-Einheit (15) und einen Kanal für die Kommunikation mit der entfernten Backend-Einheit (16).

10. Identifizierungsvorrichtung (10) nach einem der Ansprüche 1 bis 9,
wobei die Kommunikationseinheit (13) konfiguriert ist, um Näheinformationen an die nahegelegene Backend-Einheit (15) zu senden,
wobei die Näheinformationen mindestens eines von der Vorrichtungskennung (12), der persönlichen Kennung, einem Status der Identifizierungsvorrichtung (10), einem Status der Kommunikationseinheit (13), einem Status der Verbindungsqualität in Bezug auf die Kommunikation zwischen der Kommunikationseinheit (13) und der nahegelegenen Backend-Einheit (15) und/oder, im Fall der Ansprüche 6 bis 9 betreffend die entfernte Backend-Einheit (16), einem Status der Paarungseinheit (14), einer Angabe, ob die persönliche Kennung erfolgreich mit der Vorrichtungskennung (12) verknüpft wurde, oder eine beliebige Kombination davon umfasst.

11. Identifizierungsvorrichtung (10) nach einem der Ansprüche 1 bis 10,
wobei die Identifizierungsvorrichtung (10) eine Rückmeldeeinheit (17) umfasst,
wobei die Rückmeldeeinheit (17) konfiguriert ist, um eine Rückmeldung für einen Benutzer und den Träger in Bezug auf mindestens eines von der Vorrichtungskennung (12), der persönlichen Kennung, einem Status der Identifizierungsvorrichtung (10), einem Status der Kommunikationseinheit (13), einem Status der Verbindungsqualität in Bezug auf die Kommunikation zwischen der Kommunikationseinheit (13) und der nahegelegenen Backend-Einheit (15) und, im Fall der Ansprüche 6 bis 9 betreffend die entfernte Backend-Einheit (16), einem Status der Paarungseinheit (14), einer Angabe, ob die persönliche Kennung erfolgreich mit der Vorrichtungskennung (12) verknüpft wurde, oder eine beliebige Kombination davon bereitzustellen.

12. Identifizierungsvorrichtung (10) nach Anspruch 11,
wobei die Rückmeldung mindestens eine von einer optischen Rückmeldung, einer akustischen Rückmeldung, einer haptischen Rückmeldung oder eine beliebige Kombination davon umfasst, und
wobei die Rückmeldeeinheit (17) mindestens eines von einem Bildschirm oder einer Anzeige oder einem Licht, einem Lautsprecher oder einem Piepser, einer Vibrationseinheit oder einem Vibrationsmotor, oder eine beliebige Kombination davon umfasst, und
wobei die Rückmeldeeinheit (17) konfiguriert ist, um Anweisungen für einen Benutzer und/oder den Träger insbesondere in Bezug auf die Konfiguration und/oder Verwendung der Identifizierungsvorrichtung (10) bereitzustellen, und
wobei, im Fall einer Ablösung der Identifizierungsvorrichtung (10) von dem Absorptionsartikel, die Rückmeldeeinheit (17) konfiguriert ist, um einen Benutzer und/oder den Träger zu fragen und/oder zu warnen, ob diese Ablösung beabsichtigt war.

13. Identifizierungssystem (20) für einen Absorptionsartikel eines Trägers, wobei das Identifizierungssystem (20) umfasst:
eine Identifizierungsvorrichtung (10), umfassend eine Vorrichtungskennung (12), eine Kommunikationseinheit (13) und eine Paarungseinheit (14), die mit der Kommunikationseinheit (13) verbunden ist, und
eine nahegelegene Backend-Einheit (15),
wobei die Kommunikationseinheit (13) konfiguriert ist, um eine persönliche Kennung in Bezug auf den Träger von der nahegelegenen Backend-Einheit (15) zu empfangen und die persönliche Kennung für die Paarungseinheit (14) bereitzustellen, und
wobei die Paarungseinheit (14) konfiguriert ist, um die persönliche Kennung mit der Vorrichtungskennung (12) zu verknüpfen.

14. Verwendungsverfahren zur Verwendung einer Identifizierungsvorrichtung (10) für einen Absorptionsartikel eines Trägers, das Verwendungsverfahren umfassend die Schritte:
Paaren einer Identifizierungsvorrichtung (10) nach einem der Ansprüche 1 bis 12 mit einer persönlichen Kennung in Bezug auf den Träger, und
Befestigen der Identifizierungsvorrichtung (10) an dem Absorptionsartikel des Trägers.

15. Anordnung von Identifizierungsvorrichtungen für Absorptionsartikel von Trägern, die Anordnung umfassend:
mindestens zwei Identifizierungsvorrichtungen nach einem der Ansprüche 1 bis 12,
wobei jede eines Teils der mindestens zwei Identifizierungsvorrichtungen mit einer persönlichen Kennung in Bezug auf einen entsprechenden Träger gepaart und an dem jeweiligen Absorptionsartikel befestigt ist, und
wobei jede eines Teils der mindestens zwei Identifizierungsvorrichtungen entweder geladen wird oder einsatzbereit ist.

## Revendications

1. Dispositif d'identification (10) pour un article absorbant d'un porteur, le dispositif d'identification (10) comprenant :
un identifiant de dispositif (12),
une unité de communication (13) et
une unité d'appariement (14) connectée à l'unité de communication (13),
dans lequel l'unité de communication (13) est configurée pour recevoir un identifiant personnel par rapport au porteur à partir d'une unité dorsale de proximité (15) et pour fournir l'identifiant personnel pour l'unité d'appariement (14), et
dans lequel l'unité d'appariement (14) est configurée pour lier l'identifiant personnel à l'identifiant de dispositif (12).

2. Dispositif d'identification (10) selon la revendication 1,
dans lequel l'identifiant personnel est lié à l'identifiant de dispositif (12), en particulier sur le côté du dispositif d'identification (10),
dans lequel le dispositif d'identification (10) est fixé à l'article absorbant, et
dans lequel l'article absorbant comprend ou est une couche (11), et
dans lequel le dispositif d'identification (10) comprend une unité de surveillance étant configurée pour surveiller au moins un état de l'article absorbant, de préférence l'humidité et/ou les exsudats à l'intérieur de l'article absorbant, et/ou au moins un état par rapport au porteur.

3. Dispositif d'identification (10) selon la revendication 1 ou 2,
dans lequel l'identifiant de dispositif (12) est visiblement fixé au dispositif d'identification (10), et/ou
dans lequel l'identifiant de dispositif (12) comprend au moins l'un parmi un numéro de série, une étiquette (50b), un code de réponse rapide (50c), une étiquette de communication en champ proche, un numéro de série électronique, ou l'une quelconque combinaison de ceux-ci.

4. Dispositif d'identification (10) selon l'une quelconque des revendications 1 à 3,
dans lequel l'identifiant personnel comprend au moins l'un parmi un numéro d'identification associé au porteur, le nom du porteur, les initiales du porteur, le lieu de résidence du porteur, l'un quelconque autre type d'informations personnelles par rapport au porteur, ou l'une quelconque combinaison de ceux-ci.

5. Dispositif d'identification (10) selon l'une quelconque des revendications 1 à 4,
dans lequel la communication entre l'unité de communication (13) et l'unité dorsale de proximité (15) est basée sur une communication à courte distance et
dans lequel l'unité de communication (13) comprend un premier moyen de communication pour la communication courte distance et un second moyen de communication pour la communication longue distance.

6. Dispositif d'identification (10) selon l'une quelconque des revendications 1 à 5,
dans lequel l'unité de communication (13) est configurée pour communiquer avec une unité dorsale distante (16), et
dans lequel l'unité de communication (13) est configurée pour envoyer des informations à distance à l'unité dorsale distante (16),
dans lequel les informations distantes comprennent au moins l'un parmi l'identifiant de dispositif (12), un état de l'article absorbant, un état de l'humidité et des exsudats à l'intérieur de l'article absorbant, un état du dispositif d'identification (10), un état de l'unité de communication (13), un état de la qualité de connexion par rapport à la communication entre l'unité de communication (13) et l'unité dorsale de proximité (15) et/ou l'unité dorsale distante (16), un état de l'unité d'appariement (14), une indication de si l'identifiant personnel a été lié avec succès à l'identifiant de dispositif (12), ou l'une quelconque combinaison de ceux-ci.

7. Dispositif d'identification (10) selon la revendication 6,
dans lequel la communication entre l'unité de communication (13) et l'unité dorsale distante (16) est basée sur un réseau local sans fil, Bluetooth, ZigBee, ou l'un quelconque autre type de technologie de communication sans fil.

8. Dispositif d'identification (10) selon la revendication 6 ou 7,
dans lequel l'unité de communication (13) est configurée pour recevoir des données, lesdites données étant indépendantes du porteur, plus préférablement des données de configuration par rapport au dispositif d'identification (10), le plus préférablement une mise à jour de micrologiciel par rapport au dispositif d'identification (10), à partir de l'unité dorsale distante (16).

9. Dispositif d'identification (10) selon l'une quelconque des revendications 6 à 8,
dans lequel l'unité de communication (13) est configurée pour utiliser deux canaux de communication distincts comprenant un canal pour la communication avec l'unité dorsale de proximité (15) et un canal pour la communication avec l'unité dorsale distante (16).

10. Dispositif d'identification (10) selon l'une quelconque des revendications 1 à 9,
dans lequel l'unité de communication (13) est configurée pour envoyer des informations de proximité à l'unité dorsale de proximité (15),
dans lequel les informations de proximité comprennent au moins l'un parmi l'identifiant de dispositif (12), l'identifiant personnel, un état du dispositif d'identification (10), un état de l'unité de communication (13), un état de la qualité de connexion par rapport à la communication entre l'unité de communication (13) et l'unité dorsale de proximité (15) et/ou, dans le cas des revendications 6 à 9 concernant l'unité dorsale distante (16), un état de l'unité d'appariement (14), une indication de si l'identifiant personnel a été lié avec succès à l'identifiant de dispositif (12), ou l'une quelconque combinaison de ceux-ci.

11. Dispositif d'identification (10) selon l'une quelconque des revendications 1 à 10,
dans lequel le dispositif d'identification (10) comprend une unité de rétroaction (17),
dans lequel l'unité de rétroaction (17) est configurée pour fournir une rétroaction à un utilisateur, et au porteur par rapport à au moins l'un parmi l'identifiant de dispositif (12), l'identifiant personnel, un état du dispositif d'identification (10), un état de l'unité de communication (13), un état de la qualité de connexion par rapport à la communication entre l'unité de communication (13) et l'unité dorsale de proximité (15) et, dans le cas des revendications 6 à 9 concernant l'unité dorsale distante (16), un état de l'unité d'appariement (14), une indication de si l'identifiant personnel a été lié avec succès à l'identifiant de dispositif (12), ou l'une quelconque combinaison de ceux-ci.

12. Dispositif d'identification (10) selon la revendication 11,
dans lequel la rétroaction comprend au moins l'une parmi une rétroaction optique, une rétroaction acoustique, une rétroaction haptique ou l'une quelconque combinaison de celles-ci, et
dans lequel l'unité de rétroaction (17) comprend au moins l'un parmi un écran ou une unité d'affichage ou une lumière, un haut-parleur ou un bip, une unité de vibration ou un moteur de vibration, ou l'une quelconque combinaison de ceux-ci, et
dans lequel l'unité de rétroaction (17) est configurée pour fournir des instructions à un utilisateur, et/ou au porteur, en particulier par rapport à la configuration et/ou l'utilisation du dispositif d'identification (10), et
dans lequel, en cas de détachement du dispositif d'identification (10) de l'article absorbant, l'unité de rétroaction (17) est configurée pour consulter et/ou avertir un utilisateur, et/ou le porteur si ledit détachement était prévu.

13. Système d'identification (20) pour un article absorbant d'un porteur, le système d'identification (20) comprenant :
un dispositif d'identification (10) comprenant un identifiant de dispositif (12), une unité de communication (13) et une unité d'appariement (14) connectée à l'unité de communication (13), et
une unité dorsale de proximité (15),
dans lequel l'unité de communication (13) est configurée pour recevoir un identifiant personnel par rapport au porteur à partir de l'unité dorsale de proximité (15) et pour fournir l'identifiant personnel pour l'unité d'appariement (14), et
dans lequel l'unité d'appariement (14) est configurée pour lier l'identifiant personnel à l'identifiant de dispositif (12).

14. Procédé d'utilisation permettant d'utiliser un dispositif d'identification (10) pour un article absorbant d'un porteur, le procédé d'utilisation comprenant les étapes consistant à :
apparier un dispositif d'identification (10) selon l'une quelconque des revendications 1 à 12 avec un identifiant personnel par rapport au porteur, et
fixer le dispositif d'identification (10) à l'article absorbant du porteur.

15. Réseau de dispositifs d'identification pour des articles absorbants de porteurs, le réseau comprenant :
au moins deux dispositifs d'identification selon l'une quelconque des revendications 1 à 12,
dans lequel chacun parmi une partie des au moins deux dispositifs d'identification est apparié à un identifiant personnel par rapport à un porteur correspondant et fixé à l'article absorbant respectif, et
dans lequel chacun parmi une partie des au moins deux dispositifs d'identification est soit en cours de charge, soit prêt à l'emploi.
